(19) Europäisches Patentamt
European Patent Office
Office européen des brevets

(11) Publication number: **0 220 435 B1**

(12) # EUROPEAN PATENT SPECIFICATION

(45) Date of publication of patent specification: **14.11.90**

(51) Int. Cl.$^5$: **C 07 D 233/32,** C 07 B 57/00

(21) Application number: **86112335.4**

(22) Date of filing: **15.04.83**

(60) Publication number of the earlier application in accordance with Art. 76 EPC: **0 092 194**

(54) **A method for preparing optically active half esters.**

(30) Priority: **16.04.82 JP 64198/82
11.08.82 JP 140299/82
08.10.82 JP 178103/82
27.04.82 JP 71779/82
17.05.82 JP 83455/82
16.06.82 JP 104585/82**

(43) Date of publication of application:
**06.05.87 Bulletin 87/19**

(45) Publication of the grant of the patent:
**14.11.90 Bulletin 90/46**

(84) Designated Contracting States:
**CH DE FR IT LI**

(56) References cited:
**US-A-3 700 659**

**Chemical Reviews, vol. 80, no. 3, June 1980, Indiana, A. Collet et al. "Optical Resolution by Direct Crystallisation of Enanitiomer Mixtures" pages 215-230**

(73) Proprietor: **SUMITOMO CHEMICAL COMPANY, LIMITED
5-33 Kitahama 4-chome Chuo-ku
Osaka-shi Osaka (JP)**

(72) Inventor: **Ohashi, Naohito
2-1-325, Kuwata-cho
Ibaraki-shi Osaka (JP)**
Inventor: **Shimago, Kozo
No. 10-4-412, Sonehigashi-machi 2-chome
Toyonaka-shi Osaka (JP)**
Inventor: **Ikeda, Takaharu
No. 10-13-109, Makitsukadai 1-chome
Sakai-shi Osaka (JP)**
Inventor: **Ishizumi, Kikuo
No. 16-10, Uenonishi 2-chome
Toyonaka-shi Osaka (JP)**
Inventor: **Katsura, Tadashi
No. 9, Yamadahigashi 2-chome
Suita-shi Osaka (JP)**
Inventor: **Minai, Masayoshi
No. 1606-5, Harimada-cho
Moriyama-shi Shiga (JP)**

(74) Representative: **Lehn, Werner, Dipl.-Ing. et al
Hoffmann, Eitle & Partner Patentanwälte
Arabellastrasse 4
D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 220 435 B1**

Courier Press, Leamington Spa, England.

(56) References cited:
Houben-Weyl "Methoden der organischen Chemie", 4th, ed., vol. 4, part 2, "Allgemeine Chemische Methoden" 1955, Georg Thieme Verlag, Stuttgart, pages 509-511

Enantiomers Racemates and Resolutions (1981) pp. 43-48

**Description**

This invention relates to a method for preparing a (4S,5R)-1,3-dibenzyl-5-alkoxy or alkenyloxycarbonyl-2-oximidazolidine-4-carboxylic acid (Ia) and a (4R,5S)-1,3-dibenzyl-5-alkoxy or alkenyloxycarbonyl-2-oxoimidazolidine-4-carboxylic acid (Ib), each being represented by the formula (I):

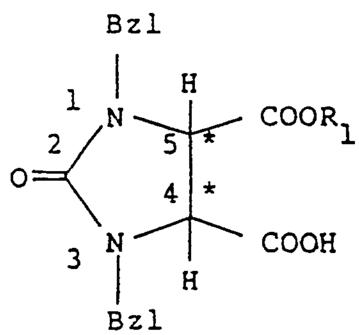

(I)

wherein $R_1$ is an alkyl group or an alkenyl group, each having 1 to 5 carbon atoms; Bzl is a benzyl group; * indicates an asymmetric carbon atom; and the 4- and 5-positions are a cis-configuration.

Another method for producing the optically half esters I is described in EP.A. 92 194.

The optically active half ester obtained by the method of this invention can be selectively reduced in either the lower alkoxycarbonyl group or carboxyl group thereof and cyclized, whereby optically active cis-1,3-dibenzylhexahydro-1H-furo[3,4-d]imidazole-2,4-dione (hereinafter abbreviated as "lactone") represented by the formula (III);

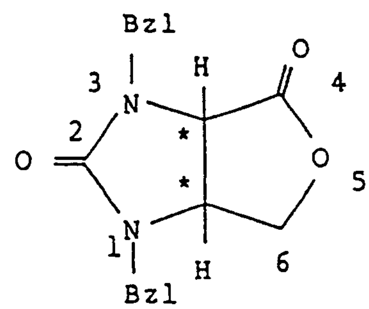

(III)

wherein Bzl is a benzyl group; * indicates an asymmetric carbon atom; and the 3a- and 6a-positions are a cis-configuration, can be produced. The lactone of the formula (III) is known as an important intermediate for the production of biotin.

In the production of the optically active lactone from the optically active half ester, the lactone produced by reducing the lower alkoxy- or alkenyloxycarbonyl group of the optically active half ester and then cyclizing and that produced by reducing the carboxyl group of the optically active half ester and then cyclizing are in reverse in respect to the configuration. In other words, when the optically active half ester having a (4S,5R)-configuration is concerned, if the lower alkoxy- or alkenyloxycarbonyl group thereof is reduced and cyclized, the resulting optically active lactone has a (3aS, 6aR)- configuration, whereas if the carboxyl group thereof is reduced and cyclized, the resulting optically active lactone has a (3aR,6aS)-configuration. On the other hand, when the optically active half ester having a (4R,5S)-configuration is concerned, if the lower alkoxy- or alkenyloxycarbonyl group thereof is reduced and cyclized, the resulting optically active lactone has a (3aR,6aS)-configuration, whereas if the carboxyl group thereof is reduced and cyclized, the resulting optically active lactone has a (3aS,6aR)-configuration.

Such is illustrated in the following scheme.

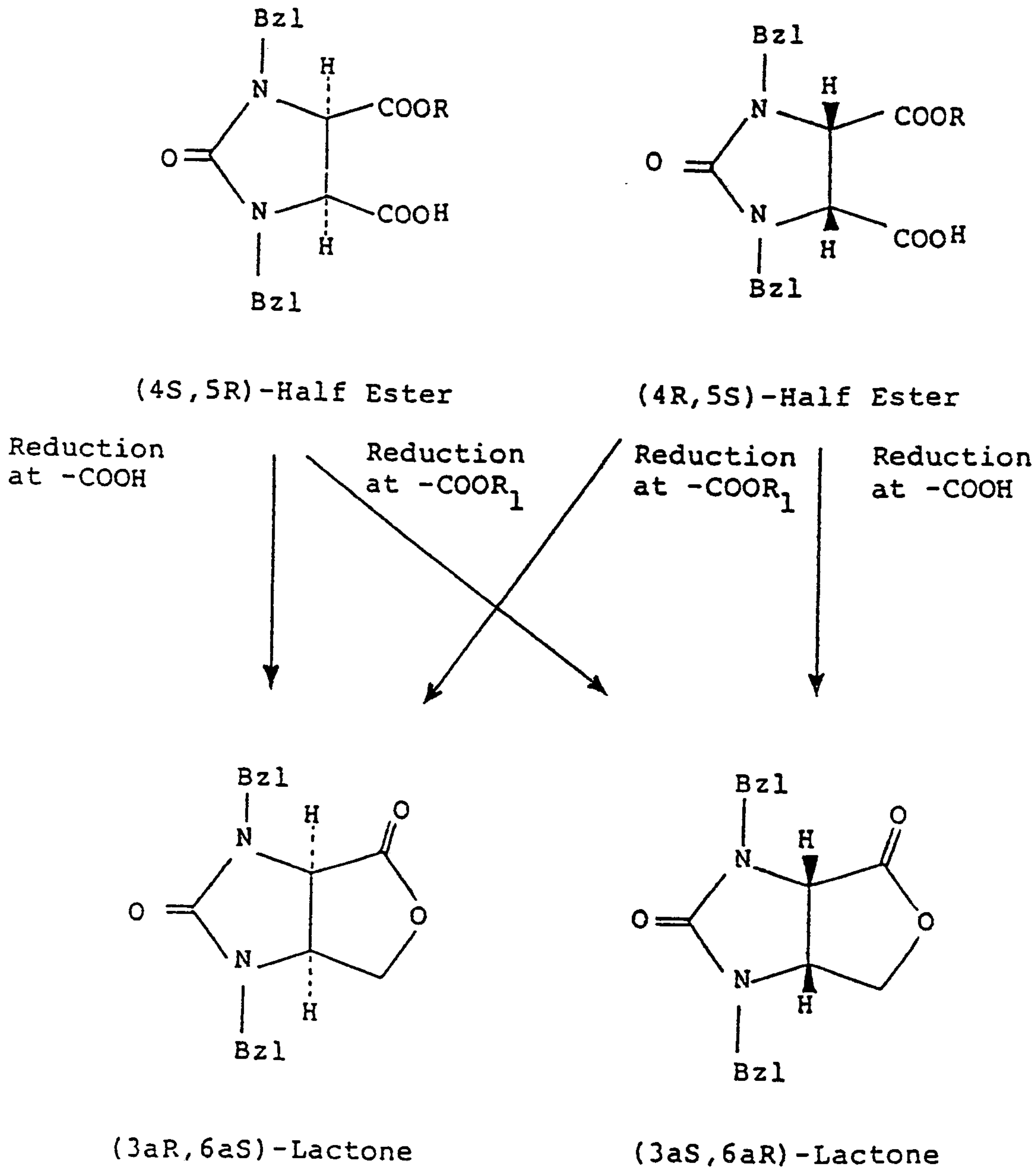


(4S,5R)-Half Ester

(4R,5S)-Half Ester

(3aR,6aS)-Lactone

(3aS,6aR)-Lactone

Accordingly, from any of the optically active (Ia) and (Ib) obtained in this invention, the optically active lactone having a (3aS,6aR)-configuration which is an important intermediate for the production of natural-type biotin can be derived. In short, any of the two optically active half esters obtained in this invention can be used as an intermediate for the production of natural-type biotin.

Such is meaningful and valuable on an industrial scale because in the usual optical resolution operation, only either one of the two optical isomers is utilized and the other is racemized and subjected to further optical resolution. Further, (Ib) is important as a starting material of a resolution agent for the production of an optically active isomer of a 4-hydroxy-2-cyclopentenone which is an intermediate for the production of prostaglandins.

The mixture of the half esters (Ia) and (Ib) can be obtained by, for example, heating a cis-1,3-dibenzyl-2-oxoimidazolidine-4,5-dicarboxylic acid and a corresponding alcohol, or by other methods (Japanese Patent Application (OPI) NBo. 172257/82). But, there have not yet been known any methods for separating the half esters (Ia) and (Ib) from the mixture thereof.

Of these half esters, only (4S,5R)-1,3-dibenzyl-5-methoxycarbonyl- or 5-n-propoxycarbonyl-2-oxo-imidazolidine-4-carboxylic acid is known by the resume of lecture for the 1982's meeting in The Agricultural Chemical Society of Japan, page 391, in more detail, *Agric. Biol. Chem.*, 46 (7), pp 1907—1910 (1982).

Such optically active compounds can be prepared by enzymatic method from cis-1,3-dibenzyl-4,5-di-methoxycarbonyl- or 4,5-di-n-propoxycarbonyl-2-oxoimidazolidine.

The invention relates to a method for the preparation of an optically active (4S,5R)-cis-1,3-dibenzyl-5-methoxy- or ethoxycarbonyl-2-oxoimidazolidine-4-carboxylic acid (Ia') or (4R,5S)-cis-1,3-dibenzyl-5-methoxy- or ethoxycarbonyl-2-oxoimidazolidine-4-carboxylic acid (Ib') comprising preferentially crystallizing (Ia') or (Ib') from a supersaturated where (Ia') and (Ib') are co-present in the presence of crystals of the same type optically active half ester as that to be crystallized.

In methods for obtaining an optically active isomer by optically resolving a racemate, a principle of a so-called preferential crystallization method in which a supersaturated solution of the racemate is seeded with a one-sided optically active isomer to separate and obtain the same type of optically active iosmer as that seeded is, in general, well known. But, it is recognized in the art that such optical resolution is advantageously applicable only to a case that a racemic mixture is formed, but that it is not applicable to a case of racemic compound. It is unclear what compound does form a racemic mixture, or whether the preferential crystallization method is applicable. Further, there are no specific regulations thereon. In other words, the case that a racemic mixture is formed to which the optical resolution is applicable is rather a specific case. Accordingly, in order to find out compounds which can meet the above described requirements, efforts must be made to a great extent. Furthermore, it is a quite rare case that a compound to be optically resolved can be subjected to preferential crystallization directly (see A. Collet et al., *Chem. Rev.*, 80, 215 (1980)).

The method of the present invention for the preparation of an optically active (4S,5R)-cis-1,3-dibenzyl-5-methoxy- or ethoxycarbonyl-2-oxoimidazolidine-4-carboxylic acid or (4R,5S)-cis-1,3-dibenzyl-5-methoxy- or ethoxycarbonyl-2-oxoimidazolidine-4-carboxylic acid, each being represented by the formula:

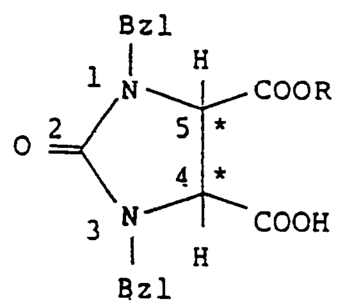

wherein R is a methyl group or an ethyl group; Bzl is a benzyl group; * indicates an asymmetric carbon atom; and the 4- and 5-positions are a cis-configuration is characterized in that an optically active (4S,5R)-cis-1,3-dibenzyl-5-methoxy- or ethoxycarbonyl-2-oxoimidazolidine-4-carboxylic acid or (4R,5S)-cis-1,3-dibenzyl-5-methoxy- or ethoxycarbonyl-2-oxoimidazolidine-4-carboxylic acid from a supersaturated solution where said optically acitve (4S,5R)-cis-1,3-dibenzyl-5-methoxy- or ethoxycarbonyl-2-oxo-imidazolidine-4-carboxylic acid and (4R,5S)-cis-1,3-dibenzyl-5-methoxy- or ethoxycarbonyl-2-oxo-imidazolidine-4-carboxylic acid are co-present in the presence of crystals of the same type of optically active (4S,5R)-cis-1,3-dibenzyl-5-methoxy- or ethoxycarbonyl-2-oxoimidazolidine-4-carboxylic acid or (4R,5S)-cis-1,3-dibenzyl-5-methoxy- or ethoxycarbonyl-2-oxoimidazolidine is preferentially crystallized.

As the method for the preparation of a supersaturated solution where the (4S,5R)-half ester and (4R,5S)-half ester are co-present, there can be illustrated usual methods for preparing supersaturated solutions, such as methods in which after heating the half esters in a suitable solvent for dissolution, the solution is cooled or concentrated, or a solvent capable of reducing the solubility of the half esters is added to the solution, and the like. The mixture of the (4S,5R)- and (4R,5S)-half esters referred to herein also includes a mixture of an original mixture (about 1:1 mixture) with a desired optically active half ester.

Illustrative examples of the solvent which can be used include alcohol solvents (e.g., methanol, ethanol, isopropyl alcohol, etc.), ketone solvents (e.g., acetone, methyl ethyl ketone, etc.), mixed solvents of these solvents and water, aromatic hydrocarbon solvents (e.g., benzene, toluene, xylene, etc.), nitrile solvents (e.g., acetonitrile, etc.), and the like. Of these solvents, benzene, toluene, xylene and acetonitrile are preferred in carrying out the optical resolution efficiently.

When the solute of the supersaturated solution where the (4S,5R)- and (4R,5S)-half esters are co-present is an optically pure racemate, it is required to add a seed crystal to the system from the outside in the resolution. On the other hand, when the solute is optically impure, i.e., a one-sided optically active isomer is present larger in amount than the other-sided optically active isomer, the former is spontaneously crystallized and hence, it is not always required to add a seed crystal to the system from the outside. In this invention, the case that the spontaneous crystallization takes place and the same function as that in seeding with a seed crystal is seen is also referred to as "seeding with a seed crystal".

There are no particular restrictions in the amount of seed crystal added, and the resolution becomes easy and accelerated as the amount of seed crystal is increased. But, it is generally sufficient to add a seed crystal in an amount of about 1 to 10% by weight based on the weight of the half ester present in the solution resolved. In a case that the objective optically active isomer is present excessively as compared with the other optically active isomer, if a seed crystal is added without taking place the spontaneous

crystallization, the amount of seed crystal can be more decreased. In view of the purposes of use, it is preferred that the optically active isomer seeded is of high purity.

The method of this invention is not limited only to a so-called one-sided resolution method in which only the one-sided optically active isomer is crystallized from the supersaturated solution. In other words, by adding seed crystals of the respective one-sided optically active isomers opposite each other to two resolution towers connected in parallel or in series, or respective portions of a resolution cell having a partition and bringing them into contact with a supersaturated solution where the (4S,5R)- and (4R,5S)-half esters are co-present, two types of optically active half esters can be obtained at the same time.

In order to increase the stability of the supersaturated solution where the (4S,5R)- and (4R,5S)-half esters are co-present, an organic base such as dimethylaniline, dimethylbenzylamine, triethylamine, etc., or an inorganic base such as sodium hydroxide, potassium hydroxide, etc. can be added in an amount of 0.05 to 0.6 mol per mol of the half esters. The addition of such base is, however, not essential.

As described above, the method of this invention can be carried out under various conditions, but one specific embodiment of the invention is set forth below.

In the optical resolution according to preferential crystallization of a racemic half ester of the formula (I) wherein R is a methyl group, it is particularly preferred to use acetonitrile as the solvent. The amount of acetonitrile is 2.5 to 5 times the weight of the racemic half ester. After the dissolution with the solvent has been carried out at 40 to 60°C, the resulting solution is cooled to 20 to 35°C to prepare a supersaturated solution. Thereafter, crystals of the desired optically active half ester are added as a seed crystal to the supersaturated solution at the same temperature, followed by stirring for 3 to 30 hours at the same temperature. Thus, preferential crystallization can be achieved.

When the lower alkoxycarbonyl group of the optically active half ester obtained by the method of this invention is selectively reduced, boron-containing reagents such as sodium boron hydride, lithium boron hydride, calcium boron hydride, etc. can be used as a reducing agent. But, it is preferred on an industrial scale to carry out the selective reduction using sodium boron hydride as the reducing agent in a mixed solvent of an alcohol solvent (e.g., ethanol, isopropyl alcohol, etc.) with an aprotic solvent (e.g., tetrahydrofuran, toluene, benzene, dichloromethane, 1,2-dichloroethane, etc.).

On the other hand, when the carboxyl group of the optically active half ester is selectively reduced, the reduction can be carried out by using a usual reducing agent capable of selectively reducing a carboxyl group, such as diborane, or by converting the carboxyl group into a corresponding active ester group or a mixed acid anhydride or acid halide with N-hydroxysuccimide, p-nitrophenol, isobutyl chloroformate, ethyl chloroformate, thionyl chloride, etc. and then reducing with a reducing agent such as sodium boron hydride.

After completion of the reduction, the reaction solution is rendered neutral or acidic and extracted with a solvent such as dichloromethane, 1,2-dichloroethane, toluene, ethyl acetate, etc. In this step, cyclization takes place whereby the optically active lactone can be obtained. In order to obtain the optically active lactone in a high yield, it is preferred to render the reaction solution after the reduction acidic.

The racemic half ester which is used as a starting material in this invention can be prepared from cis-1,3-dibenzyl-hexahydro-1H-furo[3,4-d]imidazole-2,4,6-trione and methanol or ethanol.

The optically acitive cis-1,3-dibenzyl-5-lower alkoxycarboxyl-2-oxoimidazolidine-4-carboxylic acid which is used as a seed crystal was obtained by optically resolving the racemic half ester using as a resolution agent an optically active amine selected from ephedrine and 2-amino-1,1-diphenylpropanol.

This invention is described in more detail by reference to the following examples, but it is not to be construed that the invention is limited thereto.

## Synthesis Example 1

Preparation of Racemic Half Esters:

(1) A mixture of 68.0 g of cis-1,3-dibenzyl-2-oxoimidazolidine-4,5-dicarboxylic acid anhydride, 800 ml of toluene and 12.4 g of methanol was reacted at 80 to 82°C for 3 hours and then cooled to room temperature. Crystals produced were filtered and dried under reduced pressure to obtain 61.3 g of racemic cis-1,3-dibenzyl-5-methoxycarbonyl-2-oxoimidazolidine-4-carboxylic acid. M.P., 130—131°C.

The above half ester was recrystallized from toluene to give a purified product. M.P., 131—132°C.

(2) A mixture of 70.0 g of cis-1,3-dibenzyl-2-oxoimidazolidine-4,5-dicarboxylic acid anhydride, 16.6 g of 99.5% ethanol and 1000 ml of benzene was heated under reflux for 2 hours. The reaction mixture was concentrated under atmospheric pressure. The residue was stirred overnight with 200 ml of n-hexane. Crystals produced were filtered and dried under reduced pressure to obtain 76.2 g of racemic cis-1,3-dibenzyl-5-ethoxycarbonyl-2-oxoimidazolidine-4-carboxylic acid which were then recrystallized for purification from toluene. M.P., 110—111°C.

## Synthesis Example 2

Preparation of Seed Crystals:

(1) A solution of 3.68 g of racemic cis-1,3-dibenzyl-5-methoxycarbonyl-2-oxoimidazolidine-4-carboxylic acid as prepared in Synthesis Example 1—(1) and 2.27 g of S-2-amino-1,1-diphenylpropanol in 40 ml of isopropanol was allowed to react at room temperature for 3 hours. Crystals produced were filtered to

obtain 2.16 g of a salt of (4S,5R)-cis-1,3-dibenzyl-5-methoxycarbonyl-2-oxoimidazolidine-4-carboxylic acid with S-2-amino-1,1-diphenylpropanol. M.P., 105—127°C $[\alpha]_D^{25}$ = +20.6° (c=1.0, $CH_3OH$).

2.00 g of the salt thus obtained was treated with 3% hydrochloric acid. The resulting mixture was extracted with ethyl acetate and post-treated to obtain 1.11 g of (4S,5R)-cis-1,3-dibenzyl-5-methoxycarbonyl-2-oxoimidazolidine-4-carboxylic acid. M.P., 144—146°C $[\alpha]_{365}^{25}$= −25.5° (c=1.0, DMF).

The mother liquor of the salt was concentrated under reduced pressure. The residue was treated with 3% hydrochloric acid. The resulting mixture was extracted with ethyl acetate. The extract was concentrated, and the residue was recrystallized from benzene to obtain 0.96 g of (4R,5S)-cis-1,3-dibenzyl-5-methoxycarbonyl-2-oxoimidazolidine-4-carboxylic acid. M.P., 149—150°C $[\alpha]_{365}^{25}$= + 27.2° (c=1,0, DMF).

(2) (i) To a solution of 764 mg racemic cis-1,3-dibenzyl-5-ethoxycarbonyl-2-oxoimidazolidine-4-carboxylic acid as prepared in Synthesis Example 1—(2) in 4 ml of 95% ethanol was added 310 mg l-ephedrine. After completely dissolving, the solution was allowed to stand overnight at 0 to 5°C. Crystals produced were filtered to obtain 339 mg of a salt of (4R,5S)-cis-1,3-dibenzyl-5-ethoxycarbonyl-2-oxoimidazolidine-4-carboxylic acid with l-ephedrine. M.P. 163—167°C $[\alpha]_D^{20}$ = −11.6° (c=1, $CH_3OH$).

200 mg of the salt thus obtained was treated with 20 ml of 3% hydrochloric acid. The resulting mixture was extracted with ethyl acetate and post-treated to obtain 138 mg of (4S,5R)-cis-1,3-dibenzyl-5-ethoxycarbonyl-2-oxoimidazolidine-4-carboxylic acid. M.P., 133—135°C. $[\alpha]_{365}^{25}$= +21.6° (c=1.0, DMF).

(ii) To a solution of 19.1 g of racemic cis-1,3-dibenzyl-5-ethoxycarbonyl-2-oxoimidazolidine-4-carboxylic acid as prepared in Synthesis Example 1—(2) in 200 ml of isopropanol was added 11.4 g of S-2-amino-1,1-diphenylpropanol. After completely dissolving, the solution was allowed to stand overnight at room temperature. Crystals produced were filtered to obtain 21.1 g of a salt having a melting point of 135 to 144°C. 20.0 g of the salt thus obtained was recrystallized from 500 ml of methanol to obtain 7.3 g of a salt of (4S,5R)-cis-1,3-dibenzyl-5-ethoxycarbonyl-2-oxoimidazolidine-4-carboxylic acid with S-2-amino-1,1-diphenylpropanol. M.P. 150—151°C $[\alpha]_D^{20}$ = +20.9° (c=1.0, $CH_3OH$).

6.0 g of the thus obtained salt was treated with 300 ml of 3% hydrochloric acid. The resulting mixture was extracted with ethyl acetate and post-treated to obtain 3.9 g of (4S,5R)-cis-1,3-dibenzyl-5-ethoxycarbonyl-2-oxoimidazolidine-4-carboxylic acid. M.P., 133—135°C. $[\alpha]_{365}^{25}$= −20.7° (c=1, DMF).

The above compound was recrystallized from benzene to give a purified product. M.P., 137.5—138.5°C $[\alpha]_{365}^{20}$= −21.8° (c=1, DMF).

The mother liquor of optical resolution and methanolic mother liquor were combined and concentrated under reduced pressure. The residue was treated with 800 ml of 3% hydrochloric acid. The resulting mixture was extracted with ethyl acetate. The ethyl acetate was then removed from the extract under reduced pressure. The residue was dissolved in 200 ml of isopropanol, and 8.4 g of R-2-amino-1,1-diphenylpropanol was added thereto. After completely dissolving, the solution was allowed to stand at room temperature for 6 hours. Thus, 15.0 g of crystals were produced. The crystals were recrystallized from 400 ml of methanol to obtain 8.2 g of a salt of (4R,5S)-cis-1,3-dibenzyl-5-ethoxycarbonyl-2-oxoimidazolidine-4-carboxylic acid with R-2-amino-1,1-diphenylpropanol. M.P., 150—151°C $[\alpha]_D^{20}$ = −21.0° (c=1.0, $CH_3OH$).

6.0 g of the salt thus obtained was treated with 3% hydrochloric acid, extracted with ethyl acetate and post-treated to obtain 3.7 g of (4R,5S)-cis-1,3-dibenzyl-5-ethoxycarbonyl-2-oxoimidazolidine-4-carboxylic acid. M.P., 134—146°C $[\alpha]_{365}^{20}$= +21.0° (c=1, DMF).

## Example 1

To 10.0 g of racemic cis-1,3-dibenzyl-5-methoxycarbonyl-2-oxoimidazolidine-4-carboxylic acid was added 500 ml of benzene, and the mixture was heated for dissolution. The resulting solution was gradually cooled to 52°C, and then seeded with 0.50 g of (4S,5R)-cis-1,3-dibenzyl-5-methoxycarbonyl-2-oxoimidazolidine-4-carboxylic acid, and the mixture was stirred for 2 hours while maintaining at 50 to 47°C. Crystals produced were filtered and dried to obtain 2.07 g of (4S,5R)-cis-1,3-dibenzyl-5-methoxycarbonyl-2-oxoimidazolidine-4-carboxylic acid. M.P., 148—149°C. $[\alpha]_{365}^{20}$= −27.3° (c=2, DMF). Optical purity, 98%.

To the filtrate was added 3.10 g of racemic cis-1,3-dibenzyl-5-methoxycarbonyl-2-oxoimidazolidine-4-carboxylic acid, and the mixture was heated for dissolution. The resulting solution was gradually cooled to 52°C and then seeded with 0.50 g of (4R,5S)-1,3-dibenzyl-5-methoxycarbonyl-2-oxoimidazolidine-4-carboxylic acid, and the mixture was stirred for 2 hours while maintaining at 50 to 47°C. Thus, 3.40 g of (4R,5S)-1,3-dibenzyl-5-methoxycarbonyl-2-oxoimidazolidine-4-carboxylic acid was obtained. M.P., 148—149°C $[\alpha]_{365}^{20}$= +27.0 (c=2, DMF). Optical purity, 97%.

## Example 2

To 5.0 g of racemic cis-1,3-dibenzyl-5-methoxycarbonyl-2-oxoimidazolidine-4-carboxylic acid was added 350 ml of toluene, and the mixture was heated for dissolution. The resulting solution was gradually cooled to 62°C and then seeded with 0.50 g of (4S,5R)-1,3-dibenzyl-5-methoxycarbonyl-2-oxoimidazolidine-4-carboxylic acid, and the mixture was stirred for 2 hours while maintaining at 60 to 62°C. Crystals produced were filtered and dried to obtain 1.51 g of (4S,5R)-1,3-dibenzyl-5-methoxycarbonyl-2-oxoimidazolidine-4-carboxylic acid. M.P., 147.5—148.5°C $[\alpha]_{365}^{20}$= −26.8° (c=2, DMF). Optical purity, 96%.

Example 3

To 2.00 g of racemic cis-1,3-dibenzyl-5-methoxycarbonyl-2-oxoimidazolidine-4-carboxylic acid was added 10 ml of acetonitrile, and the mixture was heated for dissolution. The resulting solution was gradually cooled to 35°C and then seeded with 0.20 g of (4S,5R)-1,3-dibenzyl-5-methoxycarbonyl-2-oxo-imidazolidine-4-carboxylic acid, and the mixture was stirred for 2 hours while maintaining at 27 to 30°C. Crystals produced were filtered and dried to obtain 0.40 g of (4S,5R)-1,3-dibenzyl-5-methoxycarbonyl-2-oxoimidazolidine-4-carboxylic acid. M.P., 143—145°C $[\alpha]_{365}^{20} = -23.9°$ (c=2, DMF). Optical purity, 86%.

Example 4

To 2.50 g of racemic cis-1,3-dibenzyl-5-methoxycarbonyl-2-oxoimidazolidine-4-carboxylic acid were added 50 ml of benzene and 70 mg of triethylamine, and the mixture was heated for dissolution. The resulting solution was gradually cooled to 50°C and then seeded with 0.25 g of (4S,5R)-1,3-dibenzyl-5-methoxycarbonyl-2-oxoimidazolidine-4-carboxylic acid, and the mixture was stirred for 2 hours while maintaining at 50 to 48°C. Crystals produced were filtered and dried to obtain 0.61 g of (4S,5R)-1,3-dibenzyl-5-methoxycarbonyl-2-oxoimidazolidine-4-carboxylic acid. M.P., 143.5—145°C $[\alpha]_{365}^{20} = -26.6°$ (c=2, DMF). Optical purity, 95%.

Example 5

To 2.50 g of racemic cis-1,3-dibenzyl-5-methoxycarbonyl-2-oxoimidazolidine-4-carboxylic acid were added 50 ml of toluene and 70 mg of triethylamine, and the mixture was heated for dissolution. The resulting solution was gradually cooled to 67°C and then seeded with 0.25 g of (4S,5R)-1,3-dibenzyl-5-methoxycarbonyl-2-oxoimidazolidine-4-carboxylic acid, and the mixture was stirred for 2 hours while maintaining at 67 to 65°C. Crystals produced were filtered and dried to obtain 0.52 g of (4S,5R)-1,3-dibenzyl-5-methoxycarbonyl-2-oxoimidazolidine-4-carboxylic acid. M.P., 145—146.5°C $[\alpha]_{365}^{20} = -27.4°$ (c=2, DMF). Optical purity, 98%.

Example 6

To 50.0 g of racemic cis-1,3-dibenzyl-5-methoxycarbonyl-2-oxoimidazolidine-4-carboxylic acid was added 200 ml of acetonitrile, and the mixture was heated at 45°C for dissolution. The resulting solution was gradually cooled to 28°C and then seeded with 0.50 g of (4S,5R)-1,3-dibenzyl-5-methoxycarbonyl-2-oxo-imidazolidine-4-carboxylic acid, and the mixture was stirred for 2 hours while maintaining at 27 to 29°C. Crystals produced were filtered and dried to obtain 5.01 g of (4S,5R)-1,3-dibenzyl-5-methoxycarbonyl-2-oxoimidazolidine-4-carboxylic acid. M.P., 145—146°C $[\alpha]_{365}^{20} = -23.4°$ (c=2, DMF). Optical purity, 84.2%.

To the filtrate were added 7.5 g of racemic 1,3-dibenzyl-5-methoxycarbonyl-2-oxoimidazolidine-4-carboxylic acid and 1.3 g of acetonitrile, and the mixture was heated as 45°C for dissolution. The resulting solution was gradually cooled to 28°C and seeded with 0.50 g of (4R,5S)-1,3-dibenzyl-5-methoxycarbonyl-2-oxoimidazolidine-4-carboxylic acid, and the mixture was stirred for 15 hours while maintaining at 27 to 29°C. Crystals produced were filtered and dried to obtain 9.38 g of (4R,5S)-1,3-dibenzyl-5-methoxycarbonyl-2-oxoimidazolidine-4-carboxylic acid. M.P., 146—147°C $[\alpha]_{365}^{23} = +25.1°$ (c=1, DMF). Optical purity, 90.3%.

Thereafter, to the filtrate from which the optically active isomer had been filtered out was added the racemic half ester, and the solution amount of the filtrate was controlled with acetonitrile. Thus, the (4S,5R)-isomer was obtained in the same manner as described above.

Such procedures were repeated. The results obtained are shown in Table 1 below.

TABLE 1

| | Racemic Half Ester | (4S,5R)-Half Ester | | | (4R,5S)-Half Ester | | |
|---|---|---|---|---|---|---|---|
| Run No. | Addition Amount (g) | Yield* (g) | Optical Purity (%) | Melting Point (°C) | Yield* (g) | Optical Purity (%) | Melting Point (°C) |
| 1 | | 4.51 | 84.2 | 145—146 | | | |
| 2 | 7.50 | | | | 8.88 | 90.3 | 146—147 |
| 3 | 7.50 | 7.57 | 86.8 | 146—147 | | | |
| 4 | 7.50 | | | | 6.46 | 91.7 | 146—147 |
| 5 | 7.50 | 5.68 | 89.1 | 146—147 | | | |
| 6 | 7.50 | | | | 5.26 | 90.9 | 146—147 |
| 7 | 7.50 | 9.23 | 86.8 | 145—146 | | | |
| 8 | 7.50 | | | | 13.27 | 93.1 | 146—147 |
| 9 | 7.50 | 5.91 | 91.7 | 146—147 | | | |
| 10 | 7.50 | | | | 1.84 | 94.6 | 146—147 |

*: The yield shown is a value from which the amount of the seed crystal (0.50 g) has been subtracted.

The filtrate obtained in Run No. 10 was concentrated under reduced pressure, and 250 ml of toluene was added to the residue to produce crystals. The crystals were filtered and dried to recover 45.3 g of racemic cis-1,3-dibenzyl-5-methoxycarbonyl-2-oxoimidazolidine-4-carboxylic acid. M.P., 129—130°C $[\alpha]^{23}_{365}$= 2.2° (c=1, DMF).

Example 7

To 10.0 g of racemic cis-1,3-dibenzyl-5-ethoxycarbonyl-2-oxoimidazolidine-4-carboxylic acid was added 100 ml of toluene, and the mixture was heated for dissolution. The resulting solution was gradually cooled to 42°C and then seeded with 0.50 g of (4S,5R)-1,3-dibenzyl-5-ethoxycarbonyl-2-oxoimidazolidine-4-carboxylic acid, and the mixture was stirred for 2 hours while maintaining at 39 to 41°C. Crystals produced were filtered and dried to obtain 1.57 g of (4S,5R)-1,3-dibenzyl-5-ethoxycarbonyl-2-oxoimidazolidine-4-carboxylic acid. M.P., 135—137°C $[\alpha]^{20}_{365}$= −20.5° (c=2, DMF). Optical purity, 94%.

To the filtrate was added 2.00 g of racemic cis-1,3-dibenzyl-5-ethoxycarbonyl-2-oxoimidazolidine-4-carboxylic acid, and the mixture was heated for dissolution. The resulting solution was gradually cooled to 42°C and then seeded with 0.50 g of (4R,5S)-1,3-dibenzyl-5-ethoxycarbonyl-2-oxoimidazolidine-4-carboxylic acid, and the mixture was stirred for 2 hours while maintaining at 39 to 41°C. Crystals produced were filtered and dried to obtain 2.23 g of (4R,5S)-1,3-dibenzyl-5-ethoxycarbonyl-2-oxoimidazolidine-4-carboxylic acid was obtained. M.P., 135.5—137.5°C $[\alpha]^{20}_{365}$= +20.9° (c=2, DMF). Optical purity, 96%.

Example 8

To 2.50 g of racemic cis-1,3-dibenzyl-5-ethoxycarbonyl-2-oxoimidazolidine-4-carboxylic acid was added 25 ml of xylene, and the mixture was heated for dissolution. The resulting solution was gradually cooled to 60°C and then seeded with 0.25 g of (4S,5R)-1,3-dibenzyl-5-methoxycarbonyl-2-oxoimidazolidine-4-carboxylic acid, and the mixture was stirred for 2 hours while maintaining at 55 to 57°C. Crystals produced were filtered and dried to obtain 0.72 g of (4S,5R)-1,3-dibenzyl-5-methoxycarbonyl-2-oxoimidazolidine-4-carboxylic acid. M.P., 117—120°C $[\alpha]^{20}_{365}$= −13.5° (c=2, DMF). Optical purity, 62%.

Reference Example 1

To a mixed solution of 320 mg of sodium boron hydride and 35 ml of isopropyl alcohol was added dropwise a solution of 1.20 g of (4S,5R)-1,3-dibenzyl-5-methoxycarbonyl-2-oxoimidazolidine-4-carboxylic acid as obtained in Example 1 dissolved in 15 ml of tetrahydrofuran at room temperature. The reaction mixture was stirred for 7 hours at 60 to 70°C. The resulting reaction mixture was cooled, and 2.5 g of concentrated hydrochloric acid was added thereto, followed by concentrating the reaction mixture under reduced pressure. 250 ml of water was added to the residue, and the mixture was extracted twice with 100 ml of chloroform. The chloroform layer was washed with water, dried over anhydrous magnesium sulfate

and concentrated under reduced pressure to obtain 1.10 g of a residue. The residue was recrystallized from hydrated isopropyl alcohol to obtain 0.90 g of (3aS,6aR)-1,3-dibenzylhexahydro-1H-furo[3,4-d]imidazole-2,4-dione. M.P., 116—118°C. $[\alpha]_D^{20} = +62.5°$ (c=1, $CHCl_3$).

Reference Example 2

To a solution of 1.84 g of (4R,5S)-1,3-dibenzyl-5-methoxycarbonyl-2-oxoimidazolidine-4-carboxylic acid as obtained in Example 1 and 15 ml of tetrahydrofuran was gradually added 226 mg of sodium boron hydride at −20°C. 1.06 g of a boron trifluoride-ether complex was then added dropwise thereto, and the mixture was stirred at the same temperature for one hour and at room temperature for 3 hours. The reaction mixture was poured into 50 ml of ice water and stirred for one hour at room temperature. The resulting reaction mixture was controlled at a pH of 9 with a potassium carbonate aqueous and extracted twice with 50 ml of ethyl acetate. The organic layer was washed with saturated salt water, dried over anhydrous magnesium sulfate and concentrated under reducing pressure. The residue was purified by silica gel column chromatography to obtain 1.10 g of (3aS,6aR)-1,3-dibenzylhexahydro-1H-furo(3,4-d)imidazole-2,4-dione. M.P., 116—118°C $[\alpha]_D^{20} = +62.2°$ (c=1, $CHCL_3$).

Reference Example 3

To a mixed solution of 660 mg of sodium boron hydride and 15 ml of isopropyl alcohol was added dropwise a solution composed of 3.68 g of (4S,5R)-1,3-dibenzyl-5-methoxycarbonyl-2-oxoimidazolidine-4-carboxylic acid as obtained in Run No. 9 of Example 6, 5 ml of isopropyl alcohol and 60 ml of 1,2-dichloroethane at 5 to 10°C over 30 minutes. After completion of the dropwise addition, the mixture was allowed to react at room temperature for 20 hours with stirring. 50 ml of 1N hydrochloric acid was added to the reaction mixture, followed by stirring at 55 to 60°C for 30 minutes. The reaction mixture was subjected to liquid-separation, and the aqueous layer was extracted once with 50 ml of 1,2-dichloroethane. The extract was combined with the organic layer and washed with water, dried over anhydrous magnesium sulfate and concentrated under reduced pressure to obtain 3.10 g of (3aS,6aR)-1,3-dibenzylhexahydro-1H-furo[3,4-d]imidazole-2,4-dione. M.P., 114—116°C $[\alpha]_D^{20} = +56.7°$ (c=1, $CHCl_3$).

Reference Example 4

To a mixed solution of 0.57 g of sodium boron hydride and 22 ml of tetrahydrofuran was added dropwise a solution of 3.40 g of a boron trifluoride-ether complex and 8 ml of tetrahydrofuran at room temperature. After completion of the dropwise addition, the mixture was stirred for 2 hours at the same temperature. To the reaction mixture was added dropwise a solution of 5.0 g of (4R,5S)-1,3-dibenzyl-5-methoxycarbonyl-2-oxoimidazolidine-4-carboxylic acid as obtained in Run No. 8 of Example 6 and 25 ml of tetrahydrofuran at room temperature. After completion of the dropwise addition, the mixture was allowed to react at the same temperature for 5 hours with stirring. 50 ml of a 5% hydrochloric acid aqueous solution was added thereto and refluxed for 30 minutes, followed by concentration such that the amount of reaction mixture was about 50 ml. The residue was extracted with 100 ml of 1,2-dichloroethane and subjected to liquid-separation. The organic layer was washed with water, dried over anhydrous magnesium sulfate and concentrated under reduced pressure to obtain 4.38 g of (3aS,6aR)-1,3-dibenzylhexahydro-1H-furo[3,4-d]imidazole-2,4-dione. M.P., 107—110°C $[\alpha]_D^{25} = +52.3°$ (c=1, $CHCl_3$).

4.00 g of crude crystals thus obtained were recrystallized from a 70% (v/v) isopropyl alcohol aqueous solution to obtain 3.18 g of purified crystals. M.P., 117—118°C $[\alpha]_D^{25} = +62.4°$ (c=1, $CHCl_3$).

Reference Example 5

To a mixture of 43 mg of lithium boron hydride and 3 ml of tetrahydrofuran was added dropwise a solution of 249 mg of (4S,5R)-1,3-dibenzyl-5-ethoxycarbonyl-2-oxoimidazolidine-4-carboxylic acid as obtained in Example 7 dissolved in 4 ml of tetrahydrofuran at room temperature. The mixture was refluxed for 2 hours, and the tetrahydrofuran was distilled off. To the residue was gradually added 4 ml of methanol under cooling, and 0.5 g of concentrated hydrochloric acid was added thereto. The mixture was refluxed for one hour and concentrated under reduced pressure. The residue was purified by silica gel chromatography to obtain 150 mg of (3aS,6aR)-1,3-dibenzylhexahydro-1H-furo[3,4-d]imidazole-2,4-dione. M.P., 114—116°C. $[\alpha]_D^{20} = +58.1°$ (c=1, $CHCl_3$).

Reference Example 6

To a solution of 382 mg of (4R,5S)-1,3-dibenzyl-5-ethoxycarbonyl-2-oxoimidazolidine-4-carboxylic acid as obtained in Example 7 and 5 ml of tetrahydrofuran was gradually added 47 mg of sodium boron hydride at −20°C. 220 mg of a boron trifluoride-ether complex was then added dropwise thereto, and the mixture was stirred at the same temperature for one hour and at room temperature for 3 hours. The reaction mixture was poured into 10 ml of ice water and stirred for one hour at room temperature. The resulting reaction mixture was controlled at a pH of 9 with a potassium carbonate aqueous solution and extracted twice with 50 ml of ethyl acetate. The organic layer was washed with saturated salt water, dried over anhydrous magnesium sulfate and concentrated under reduced pressure. The residue was purified by silica gel column chromatography to obtain 208 mg of (3aS,6aR)-1,3-dibenzylhexahydro-1H-furo[3,4-d]imidazole-2,4-dione. M.P., 114—117°C. $[\alpha]_D^{20} = +58.9°$ (c=1, $CHCl_3$).

**Claims**

1. A method for the preparation of an optically active (4S,5R)-cis-1,3-dibenzyl-5-methoxy- or ethoxycarbonyl-2-oxoimidazolidine-4-carboxylic acid or (4R,5S)-cis-1,3-dibenzyl-5-methoxy- or ethoxycarbonyl-2-oxoimidazolidine-4-carboxylic acid, each being represented by the formula:

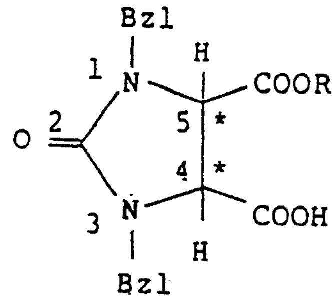

wherein R is a methyl group or an ethyl group; Bzl is a benzyl group; * indicates an asymmetric carbon atom; and the 4- and 5-positions are a cis-configuration, comprising preferentially crystallizing an optically active (4S,5R)-cis-1,3-dibenzyl-5-methoxy- or ethoxycarbonyl-2-oxoimidazolidine-4-carboxylic acid or (4R,5S)-cis-1,3-dibenzyl-5-methoxy- or ethoxycarbonyl-2-oxoimidazolidine-4-carboxylic acid from a supersaturated solution where said optically active (4S,5R)-cis-1,3-dibenzyl-5-methoxy- or ethoxycarbonyl-2-oxoimidazolidine-4-carboxylic acid and (4R,5S)-cis-1,3-dibenzyl-5-methoxy- or ethoxycarbonyl-2-oxoimidazolidine-4-carboxylic acid are co-present in the presence of crystals of the same type of optically active (4S,5R)-cis-1,3-dibenzyl-5-methoxy- or ethoxycarbonyl-2-oxoimidazolidine-4-carboxylic acid or (4R,5S)-cis-1,3-dibenzyl-5-methoxy- or ethoxycarbonyl-2-oxoimidazolidine, -4-carboxylic acid.

2. A method according to Claim 1, wherein R is a methyl group.

3. A method according to Claim 1, wherein R is an ethyl group.

4. A method according to Claim 1, wherein said supersaturated solution is prepared in a solvent selected from benzene, toluene, xylene and acetonitrile.

5. A method according to Claim 1, wherein an organic or inorganic base is added to said supersaturated solution in an amount of 0.05 to 0.6 mol per mole of the optically active (4S,5R)-cis-1,3-dibenzyl-5-methoxy- or ethoxycarbonyl-2-oxoimidazolidine-4-carboxylic acid and (4R,5S)-cis-1,3-dibenzyl-5-methoxy- or ethoxycarbonyl-2-oxoimidazolidine-4-carboxylic acid.

6. A method according to Claim 2, wherein said supersaturated solution is prepared by dissolving a racemic cis-1,3-dibenzyl-5-methoxy- or ethoxycarbonyl-2-oxoimidazolidine-4-carboxylic acid in acetonitrile at 40 to 60°C and cooling to 20 to 35°C.

**Patentansprüche**

1. Verfahren zur Herstellung einer optisch aktiven (4S,5R)-cis-1,3-Dibenzyl-5-methoxy- oder ethoxycarbonyl-2-oxoimidazolidin-4-carbonsäure oder (4R,5S)-cis-1,3-Dibenzyl-5-methoxy- oder ethoxycarbonyl-2, oxoimidazolidin-4-carbonsäure, die jeweils die Formel

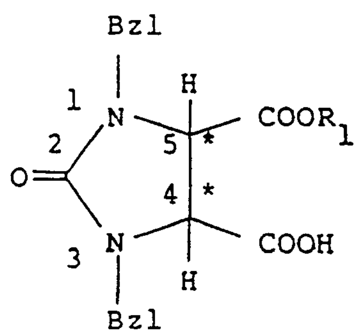

(I)

haben, wobei R eine Methylgruppe oder eine Ethylgruppe ist; Bzl eine Benzylgruppe ist; * eine asymmetrisches Kohlenstoffatom bedeutet, und die 4- oder 5-Stellungen cis-Konfigurationen sind, umfaßend das bevorzugte Kristallisieren einer optisch aktiven (4S,5R)-cis-1,3-Dibenzyl-5-methoxy- oder ethoxycarbonyl-2-oxoimidazolidin-4-carbonsäure oder (4R,5S)-cis-1,3-Dibenzyl-5-methoxy- oder ethoxycarbonyl-2-oxoimidazolidin-4-carbonsäure aus einer übersättigten Lösung, in welcher die optisch aktive (4S,5R)-cis-1,3-Dibenzyl-5-methoxy- oder ethoxycarbonyl-2, oxoimidazolidin-4-carbonsäure und (4R,5S)-cis-1,3-Dibenzyl-5-methoxy- oder ethoxycarbonyl-2-oxoimidazolidin-4-carbonsäure zusammen vorliegen

in Gegenwart von Kristallen des gleichen Types von optisch aktiver (4S,5R)-cis-1,3-Dibenzyl-5-methoxy- oder enthoxycarbonyl-2-oxoimidazolidin-4-carbonsäure oder (4R,5S)-cis-1,3-Dibenzyl-5-methoxy- oder ethoxycarbonyl-2-oxoimidazolidin-4-carbonsäure.

2. Verfahren gemäß Anspruch 1, bei dem R eine Methylgruppe ist.

3. Verfahren gemäß Anspruch 1, bei dem R eine Ethylgruppe ist.

4. Verfahren gemäß Anspruch 1, bei dem die übersättigte Lösung in einem Lösungsmittel, ausgewählt aus Benzol, Toluol, Xylol und Acetonitril hergestellt ist.

5. Verfahren gemäß Anspruch 1, bei dem eine organische oder anorganische Base zu der übersättigten Lösung in einer Menge von 0,05 bis 0,6 Mol pro Mol der optisch aktiven (4S,5R)-cis-1,3-Dibenzyl-5-methoxy- oder ethoxycarbonyl-2-oxoimidazolidin-4-carbonsäure und (4R,5S)-cis-1,3-Dibenzyl-5-methoxy- oder ethoxycarbonyl-2-oxoimidazolidin-4-carbonsäure zugegeben wird.

6. Verfahren gemäß Anspruch 2, bei dem die übersättigte Lösung hergestellt wird, indem man eine racemische cis-1,3-Dibenzyl-5-methoxy- oder ethoxycarbonyl-2-oxoimidazolidin-4-carbonsäure in Acetonitril bei 40 bis 60°C auflöst und dann auf 20 bis 35°C kühlt.

**Revendications**

1. Un procédé pour la fabrication d'un acide (4S,5R)-cis-1,3-dibenzyl-5-méthoxy ou éthoxy)carbonyl-2-oxoimidazolidine-4-carboxylique ou d'un acide (4R,5S)-cis-1,3-dibenzyl-5-méthoxy ou éthoxy)carbonyl-2-oxoimidazolidine-4-carboxylique optiquement actif, représentés chacun par la formule:

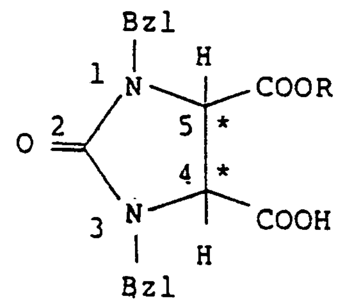

dans laquelle R est un groupe méthyle ou éthyle; Bzl est un groupe benzyl; * indique un atome de carbone asymétrique; et les atomes de carbone en positions 4 et 5 sont dans la configuration cis, comprenant la cristallisation préférentielle d'un acide (4S,5R)-cis-1,3-dibenzyl-5-méthoxy ou éthoxy)carbonyl-2-oxo-imidazolidine-4-carboxylique ou d'un acide (4R,5S)-cis-1,3-dibenzyl-5-méthoxy ou éthoxy)carbonyl-2-oxo-imidazolidine-4-carboxylique optiquement actif à partir d'une solution sursaturée contenant à la fois ledit acide (4S,5R)-cis-1,3-dibenzyl-5-méthoxy ou éthoxy)carbonyl-2-oxoimidazolidine-4-carboxylique et ledit acide (4R,5S)-cis-1,3-dibenzyl-5-méthoxy ou éthoxy)carbonyl-2-oxoimidazolidine-4-carboxylique optiquement actifs en présence de cristaux du méme type d'acide (4S,5R)-cis-1,3-dibenzyl-5-méthoxy ou éthoxy)carbonyl-2-oxoimidazolidine-4-carboxylique ou d'acide (4R,5S)-cis-1,3-dibenzyl-5-méthoxy ou éthoxy)carbonyl-2-oxoimidazolidine-4-carboxylique optiquement actif.

2. Un procédé selon la revendication 1, dans lequel R est un groupe méthyle.

3. Un procédé selon la revendication 1, dans lequel R est un groupe éthyle.

4. Un procédé selon la revendication 1, dans lequel ladite solution sursaturée est préparée dans un solvant choisi parmi le benzène, le toluène, le xylène et l'acétonitrile.

5. Un procédé selon la revendication 1, dans lequel on ajoute à ladite solution sursaturée une base organique ou inorganique en quantité de 0,05 à 0,6 mol par mole de l'acide (4S,5R)-cis-1,3-dibenzyl-5-méthoxy ou éthoxy)carbonyl-2-oxoimidazolidine-4-carboxylique et de l'acide (4R,5S)-cis-1,3-dibenzyl-5-méthoxy ou éthoxy)carbonyl-2-oxoimidazolidine-4-carboxylique optiquement actifs.

6. Un procédé selon la revendication 2, dans lequel ladite solution sursaturée est préparée en dissolvant un acide cis-1,3-dibenzyl-5-(méthoxy ou éthoxy)carbonyl-2-oxoimidazolidine-4-carboxylique racémique dans l'acétonitrile à 40—60°C et en refroidissant à 20—35°C.